# EUROPEAN PATENT SPECIFICATION

(11) **EP 2 527 356 B1**
(45) Date of publication and mention of the grant of the patent: **03.01.2018**
(21) Application number: 12165450.3
(22) Date of filing: 20.07.2006
(51) Int. Cl.: C07J 53/00

(54) **Process for the Production of 3-Oxo-pregn-4-ene-21,17-carbolactones by the Metal-Free Oxidation of 17-(3-Hydroxypropyl)-3,17-dihydroxyandrostanes**
Verfahren zur Herstellung von 3-Oxo-pregn-4-ene-21,17-carbolactonen mit metallfreier Oxidation von 17-(3-Hydroxypropyl)-3,17-dihydroxyandrostanen
Procédé pour la production de 3-oxo-pregn-4-ene-21,17-carbolactones par oxydation sans métal de 17-(3-hydroxypropyl)-3,17-dihydroxyandrostanes

(30) Priority: 21.07.2005 EP 05090214; 21.07.2005 US 185984
(43) Date of publication of application: 28.11.2012
(62) Divisional of application: 06762786.9
(73) Proprietor: Bayer Intellectual Property GmbH, 40789 Monheim am Rhein (DE)
(72) Inventor: Seba, Hartmut, 59423 Unna (DE); Seilz, Carsten, 59199 Bönen (DE)
(74) Representative: BIP Patents

(56) References cited:
- EP-A- 1 571 153
- WO-A-98/06738
- WO-A1-2006/061309
- US-A1- 2004 220 158
- BITTLER D ET AL: "SYNTHESIS OF SPIRORENONE - A NOVEL HIGHLY ACTIVE ALDOSTERONE ANTAGONIST", ANGEWANDTE CHEMIE. INTERNATIONAL EDITION, WILEY VCH VERLAG, WEINHEIM, DE, vol. 21, no. 9, 1982, pages 696-697, XP002047531, ISSN: 1433-7851
- NICKISCH K ET AL: "ALDOSTERONE ANTAGONISTS 2. NEW 7-ALPHA ACETYLTHIO-15 16-METHYLENESPIROLACTONES", JOURNAL OF MEDICINAL CHEMISTRY, vol. 30, no. 8, 1987, pages 1403-1409, XP002367543, ISSN: 0022-2623
- ZHANG ET AL: "Asymmetric Dihydroxylation-Haloetherification Strategy for the Synthesis of Tetrahydrofuran-Containing Acetogenins", JOURNAL OF ORGANIC CHEMISTRY, AMERICAN CHEMICAL SOCIETY. EASTON, US, vol. 63, no. 6, 1998, pages 2049-2052, XP002104971, ISSN: 0022-3263
- ANELLI P L ET AL: "OXIDATION OF DIOLS WITH ALKALI HYPOCHLORITES CATALYZED BY OXAMMONIUM SALTS UNDER TWO-PHASE CONDITIONS", JOURNAL OF ORGANIC CHEMISTRY, AMERICAN CHEMICAL SOCIETY. EASTON, US, vol. 54, no. 12, 9 June 1989 (1989-06-09), pages 2970-2972, XP000025425, ISSN: 0022-3263

## Description

The present description describes certain subject matter which does not fall within the scope of the present claims. Such subject matter is nonetheless relevant in the sense that it assists the skilled person by further illustrating technical subject matter which may help him or her to execute the claimed process. Consequently, whilst this subject matter is not embraced by the current claims and does not form part of the invention protected by the present claims, it has nonetheless been left in the present description for the purposes of better enabling the skilled person to carry out that subject matter of the presently claimed invention.

This invention relates to processes for the production of 3-oxo-pregnane-21,17-carbo-lactones as well as 3-oxo-pregn-4-ene-21,17-carbolactones, in particular processes for the production of 3-oxo-17a-pregnane-21,17-carbolactones as well as 3-oxo-17α-pregn-4-ene-21,17-carbolactones. In addition, the invention relates to the dichloromethane hemisolvate of 6β,7β;15β,16β-dimethylene-3-oxo-17α-pregnan-5β-ol-21,17 carbolactone.

Examples of pharmacologically active steroid-21,17-carbolactones are eplerenone (9α,11αa-epoxy-7α-methoxycarbonyl-3-oxo-17α-pregn-4-ene-21,17-carbolactone), drospirenone (6β,7α;15α,16α-dimethylene-3-oxo-17α-pregn-4-ene-21,17-carbolactone), spironolactone (7α-acylthio-3-oxo-17α-pregn-4-ene-21,17-carbolactone), canrenone (3-oxo-17α-pregna-4,6-diene-21,17-carbolactone), and prorenone (6β,7β-methylene-3-oxo-17α-pregna-4,6-diene-21,17-carbolactone).
The build-up of the steroid-21,17-spirolactone can be carried out by oxidation of the corresponding 17-hydroxy-17-(3-hydroxypropyl) steroid 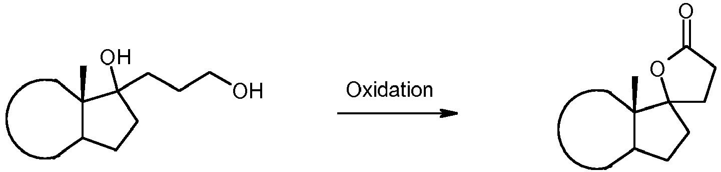 with suitable oxidizing agents such as chromic acid (Sam et al. J. Med. Chem. 1995, 38, 4518-4528), pyridinium chlorochromate (EP 075189), pyridinium dichromate (Bittler et al; Angew. Chem. [Applied Chem.] 1982, 94, 718-719; Nickisch et al. Liebigs Ann. Chem. 1988, 579-584), or potassium bromate in the presence of a ruthenium catalyst (EP 918791). The clearly pronounced formation of by-products by a number of secondary reactions is disadvantageous in the oxidation process of the prior art with chromium(VI) derivatives, by which the isolation of the pure product is hampered and the yield is reduced. The by-product profile is improved namely by the ruthenium-catalyzed oxidation (EP 918791), and thus also the yield increases. The use of transition metals in the production of pharmaceutical active ingredients, however, is generally associated with the drawback that the removal of heavy metal traces is already connected with an elevated expense. Moreover, large amounts of heavy metal-containing wastes accumulate in the production, and said wastes can be removed only in an intensive and costly way.

The object of this invention therefore consists in making available an alternate process for the production of 3-oxo-pregnane-21,17-carbolactones as well as 3-oxo-pregn-4-ene-21,17-carbolactones from the corresponding 17-(3-hydroxypropyl)-3,17-dihydroxy-androstanes that makes it possible to produce the target compounds with a higher yield and purity.
This object was achieved according to the invention in that the 17-(3-hydroxypropyl)-3,17-dihydroxyandrostanes of general formula I 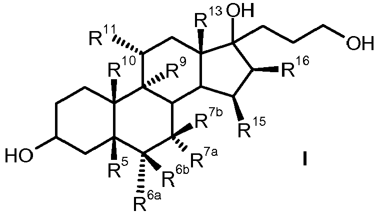 in which
- R⁵: is hydrogen, hydroxy;
- R^{6a}: is hydrogen, together with R⁵ a double bond, or together with R^{7a} a -CH₂ group;
- R^{6b}: is hydrogen, together with R^{7b} a -CH₂ group or a double bond;
- R^{7a}: is hydrogen, C₁-C₄-alkyl, C₁-C₄-alkoxycarbonyl, C₁-C₄-thioacyl or together with R^{6a} a -CH₂ group;
- R^{7b}: is hydrogen, or together with R^{6b} a -CH₂ group;
- R⁹: is hydrogen, together with R¹¹ a double bond, or together with R¹¹ an epoxy group -O-;
- R¹⁰: is hydrogen, methyl, or ethyl;
- R¹¹: is hydrogen, together with R⁹ a double bond or together with R⁹ an epoxy group -O-;
- R¹³: is hydrogen, methyl or ethyl;
- R¹⁵: is hydrogen, C₁-C₄-alkyl, together with R¹⁶ a -CH₂ group or a double bond,
- R¹⁶: is hydrogen, together with R¹⁵ a -CH₂ group or a double bond,
are reacted with an organic or inorganic hypochlorite as an oxidizing agent in the presence of catalytic amounts of a 2,2,6,6-tetramethylpiperidine-N-oxide derivative to form the 3-oxo-pregnane-21,17-carbolactones of Formula II 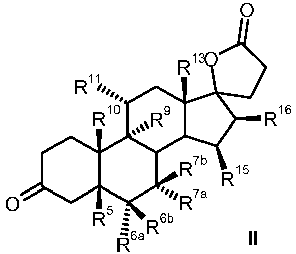 If R⁵ is a hydroxy group, the compounds of formula II can be converted in the presence of an acid at pH < 5 with water being eliminated into compounds of formula III

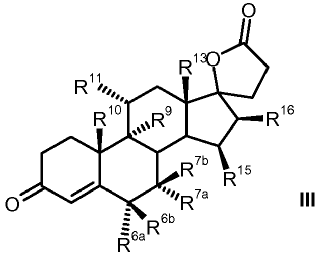

Metal-free oxidations of alcohols to the corresponding aldehydes, ketones, carboxylic acids, lactols, and lactones are collectively referred to in the survey article of W. Adam et al., Chem. Rev. 2001, 101, 3499-3548. Metal-free oxidations in the presence of 2,2,6,6-tetramehylpiperidine-N-oxide (TEMPO) are described by van Bekkum et al. in Synthesis 1996, 1153-1174.

Primary alcohols can be oxidized to aldehydes with sodium bromite (NaBrO₂) or calcium hypochlorite [Ca(OCl₂)] in the presence of TEMPO derivatives [S. Torii et al. J. Org. Chem. 1990, 55, 462-466]. Sodium hypochlorite (NaOCl) can also be used as an oxidizing agent (Org. Synth. 69, 212).
The oxidation of secondary alcohols to ketones and in particular the oxidation of primary alcohols to carboxylic acids (or with suitable diols to lactones) requires a co-catalyst (P. L. Anelli et al., J. Org. Chem. 1987, 52, 2559-2562). As a co-catalyst, a bromide (generally KBr or NaBr) is used. The addition of bromide ions can be useful even in the oxidation of primary alcohols to aldehydes (P. L. Anelli et al., J. Org. Chem. 1987, 52, 2559-2562).
The danger of the formation of bromine-containing by-products under oxidative conditions is disadvantageous in the use of bromides as co-catalysts. This oxidation method is especially suitable for the oxidation of primary alcohols to the corresponding aldehydes.
Without the addition of bromide, the TEMPO-catalyzed oxidation of secondary alcohols to the corresponding ketones requires higher excesses of hypochlorite [3-4 molar equivalents of Ca(OCl)₂, thus 6-8 molar equivalents of OCl⁻; (S. Tori et al. J. Org. Chem. 1990, 55, 462-466)].
The oxidative lactonization of 1,4-diols proceeds in many stages via the aldehyde, which first forms lactol in an intermediate stage; the quasi-secondary hydroxy group of said lactol must then be further oxidized. The oxidative lactonization of 1,4-diols therefore requires still harder conditions (at least equimolar amounts of the TEMPO derivative (J. M. Bobbitt et al., J. Org. Chem. 1991, 56, 6110-6114) or other oxidizing agents in connection with increased amounts of the TEMPO catalyst (J. Einhorn, J. Org. Chem. 1996, 61, 7452-7454; in the presence of a bromide addition: S. D. Rychnovsky, J. Org. Chem. 1999, 64, 310-312; in the presence of bromide ions produced in situ from the oxidizing agent sodium bromite: S. Torii, J. Org. Chem. 1990, 55, 462-466). In view of the prior art, it was therefore surprising that oxidative lactonization on the D-ring and the oxidation of the secondary 3-hydroxy group of the 17-(3-hydroxypropyl)-3,17-dihydroxyandrostanes of general formula I (altogether three oxidation stages) can be performed successfully at the same time under mild conditions in the presence of catalytic amounts of TEMPO derivatives. In addition, it was surprising that the process according to the invention can be performed with only 1.0 to 2.0 equivalents of hypochlorite per oxidation stage, thus altogether 3.0 to 6.0 molar equivalents of hypochlorite quite without the co-catalytic bromide additions. WO-A-98/06738 discloses the oxidative lactonisation producing the same compounds as produced in the instant invention, using NaBrO₃ as oxidant and RuCl₃ as catalyst.

The process according to the invention is performed with a total of at least 3 molar equivalents of alkali hypochlorite, organic hypochlorite or at least 1.5 molar equivalents of alkaline-earth hypochlorite as oxidizing agent; preferably with 3-6 molar equivalents of alkali hypochlorite, or 1.5-3 molar equivalents of alkaline-earth hypochlorite, especially preferably 3-4 molar equivalents of alkali hypochlorite or 1.5-2 molar equivalents of alkaline-earth hypochlorite.

The concentration of the aqueous hypochlorite solution during the oxidation is preferably adjusted such that it is 0.8 to 1.1 mol of hypochlorite/kg.
Sodium hypochlorite, potassium hypochlorite, calcium hypochlorite or tert-butyl hypochlorite are preferably used as oxidizing agents.
The 2,2,6,6-tetramethylpiperidine-N-oxide derivatives (TEMPO derivatives) are used in catalytic amounts, whereby the amount is preferably 1-5 mol%, especially preferably 1-1.5 mol%.
Suitable TEMPO derivatives are, i.a., the 2,2,6,6-tetramethylpiperidine-N-oxide (TEMPO), the 4-methoxy-2,2,6,6-tetramethylpiperidine-N-oxide (4-MeO-TEMPO) as well as the 4-benzyloxy-2,2,6,6-tetramethylpiperidine-N-oxide (4-BnO-TEMPO). TEMPO is preferably used according to this invention, especially preferably in an amount of 1-5 mol%, quite especially preferably 1-1.5 mol%.
The oxidation is carried out in an organic solvent or in a two-phase solvent-water mixture, whereby the solvent is selected such that both the TEMPO derivative and the compounds of formula I can be well dissolved therein.
The reaction is preferably performed in a two-phase system. The process according to the invention is quite preferably performed in a dichloromethane-water mixture.

The oxidation is performed according to the invention at a temperature of 0 to 20°C, preferably at 10-20°C.
During the oxidation, the pH of the reaction solution is to be at least 8.0; preferably 8.5 to 10.0; especially preferably 9.0 to 9.5.
The pH can suitably be adjusted with a suitable Brönsted acid, such as organic acids (e.g., acetic acid) or inorganic acids (HCl, H₂SO₄, H₃PO₄) or acid salts of multivalent acids (bicarbonates, hydrogen sulfates, hydrogen phosphates, etc.). Alkali bicarbonates, especially preferably potassium bicarbonate, are preferably used.
The oxidation reaction is brought to a halt by adding a reducing agent to quench excess hypochlorite reagent. For this purpose, any reducing agent with corresponding redox potential that is known to one skilled in the art is suitable. An aqueous alkali hydrogen sulfite solution is preferably used according to this invention. Sodium or potassium hydrogen sulfite (NaHSO₃ or KHSO₃), the aqueous solution of sodium or potassium disulfite (Na₂SO₂O₅ or K₂S₂O₅) is especially preferably used.

If, in the reaction mixture, the excess hypochlorite reagent at pH < 5 is quenched thus without the addition of a base or a basic buffer, or in the presence of a further acid addition, the 3-oxo-pregnane-21,17-carbolactones of formula II (if R⁵ = OH) thus eliminate water, and equally the 3-oxo-pregn-4-ene-21,17-carbolactones of formula III are formed in the reaction mixture. The completion of the oxidation reaction at a pH of less than 5 makes possible the production of compounds of formula III in a one-pot process.
If, in the reaction mixture, the excess hypochorite reagent is quenched with the addition of a base or a basic buffer at pH > 5, the 3-oxo-pregnane-21,17-carbolactones of formuIa II can be isolated. The completion of the oxidation reaction at a pH of more than 5 makes possible the specific production of compounds of formula II.
Since in the case R⁵ = OH the solubility of the compounds of formula II in comparison to the compounds of formula III in organic solvents is lower, the specific isolation of the compounds of formula II as an intermediate on the path to compounds of formula III offers the special advantage of the possibility of a more effective purification (e.g., by crystallization). The purified intermediates can be reacted according to the methods that are known in the literature with a suitable acid (such as, e.g., sulfuric acid, hydrochloric acid, para-toluenesulfonic acid, etc.) to form compounds of formula III (EP 0918791).

To adjust the pH, any suitable inorganic or organic base or any suitable buffer or any suitable buffer system can be used. The base or buffer is preferably added mixed or in parallel to the reaction mixture with the reducing agent.
According to this invention, sodium phosphate (Na₃PO₄) is preferably used as a basic buffer.
17-(3-Hydroxypropyl)-3,17-dihydroxyandrostanes of general formula I can be obtained, e.g., starting from the correspondingly substituted 3-hydroxy-17-ketoandrostanes by the addition of propargyl alcohol at C-17 and subsequent hydrogenation of the triple bond (EP 918791, EP 51143, DE 3026783) or as described by N. W. Atwater in J. Org. Chem. 1961, 26, 3077 and in US 4,069,219 or in the documents cited therein.
The corresponding 3-hydroxy-17-ketoandrostanes can be produced in turn from the correspondingly substituted 3-hydroxyandrost-5-en-17-one (EP 51143, DE 3026783).

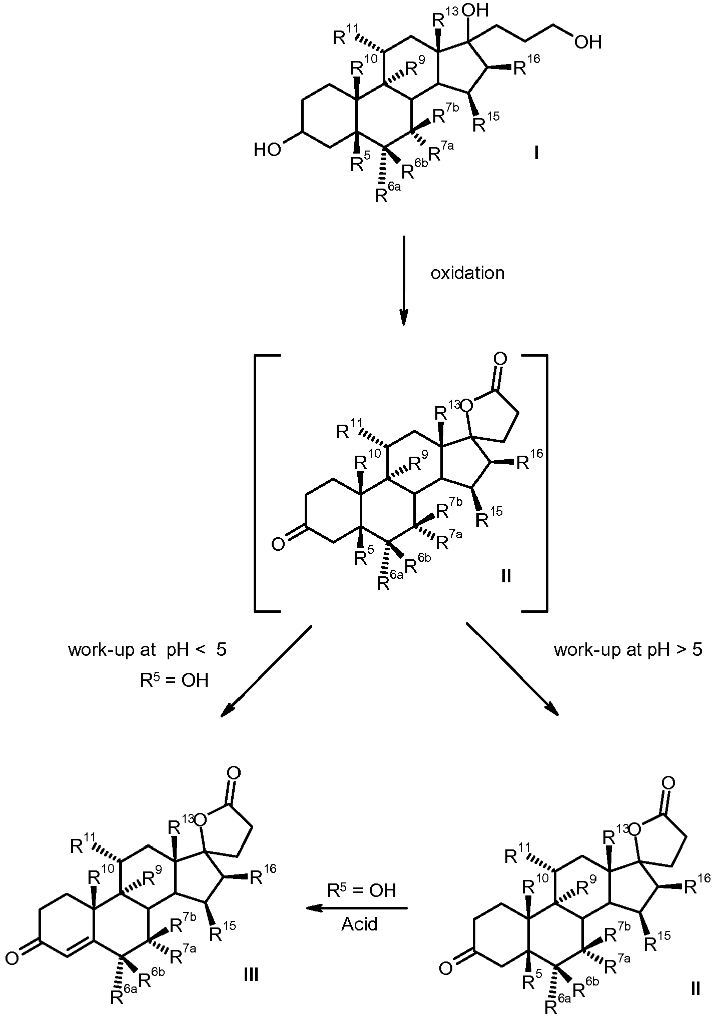

The process according to the invention is suitable especially for the production of 3-oxo-17α-pregnane-21,17-carbolactones of formula IIa 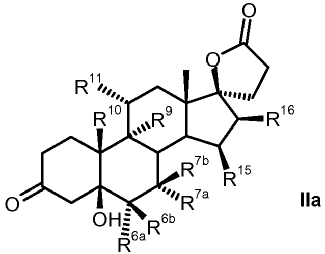 as well as 3-oxo-17α-pregn-4-ene-21,17-carbolactones of formula IIIa, 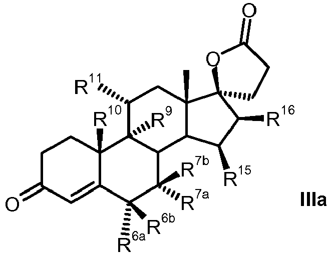 in which the substituents R have the following meaning:
- R^{6a}: is hydrogen or together with R^{7a} a -CH₂ group;
- R^{6b}: is hydrogen, together with R^{7b} a -CH₂ group, or a double bond;
- R^{7a}: is hydrogen, C₁-C₄-alkoxycarbonyl, or C₁-C₄-thioacyl;
- R^{7b}: is hydrogen, or together with R^{6b} a -CH₂ group,
- R⁹: is hydrogen, together with R¹¹ a double bond or together with R¹¹ an epoxy group -O-;
- R¹⁰: is hydrogen, or methyl;
- R¹¹: is hydrogen, together with R⁹ a double bond or together with R⁹ an epoxy group -O-;
- R¹⁵: is hydrogen, together with R¹⁶ a -CH₂ group or a double bond;
- R¹⁶: is hydrogen, together with R¹⁵ a -CH₂ group or a double bond;
whereby as starting materials, the 17-(3-hydroxypropyl)-3,17-dihydroxyandrostanes of general formula Ia 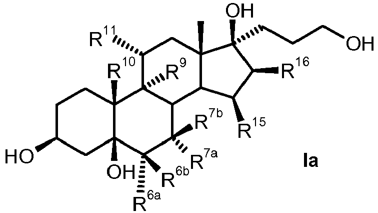 are used.

The process according to the invention for the production of compounds of formulas IIa and IIIa,
in which
- R^{6a}, R^{7a}, R⁹, R¹¹: are hydrogen;
- R^{6b} and R^{7b}: together are a -CH₂ group;
- R¹⁰: is methyl;
- R¹⁵ and R¹⁶: together are a -CH₂ group;
thus compounds IIb as well as IIIb, whereby the compound of formula Ib is used as a starting material is quite especially suitable.

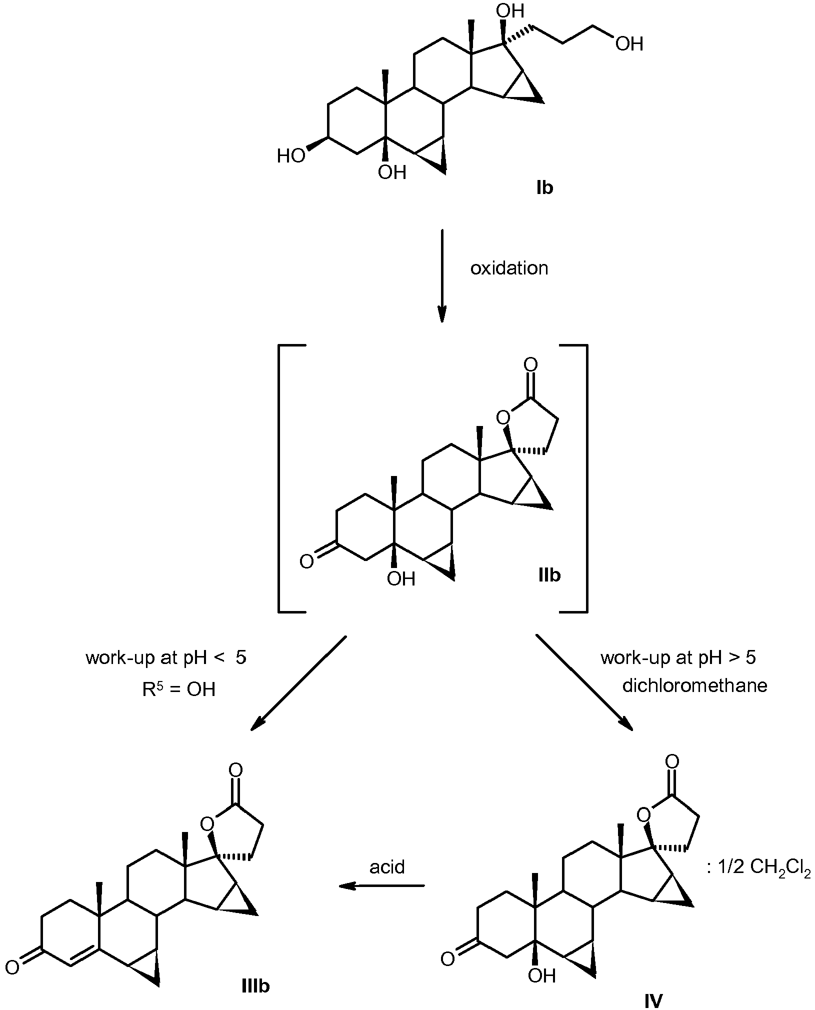

Another aspect of this invention is the poorly soluble dichloromethane-hemisolvate IV that is formed, surprisingly enough, from compound IIb when the process according to the invention is performed in dichloromethane and is worked up, basic, at pH > 5. During the oxidation, this poorly soluble product precipitates, and thus the influence of the oxidizing agent and thus possible further reactions, which can result in the formation of by-products, are evaded.

The dichloromethane-hemisolvate IV is distinguished by a strict and constant melting point, which is 121 °C, while compound IIb melts at 188°C. DSC (Differential Scanning Calorimetry) measurements have shown that compound IV is stable up to the melting point.
After the reaction is completed, the precipitation of compound IV from the reaction solution by adding a non-polar solvent, preferably an ether, especially preferably diisopropyl ether, is completed. The non-polar oxidation and elimination products that are produced with the oxidation remain largely dissolved in the ether-dichloromethane mixture, which makes possible an extremely slight isolation of the compound IV at a high purity.
In this way, compound IV with a yield of 82% is obtained. The thus obtained product contains no more than 6% steroidal contaminants and can easily be reacted without further purification according to known methods with a suitable acid to form drospirenone IIIb (EP 918791). The synthesis variant that runs through the isolated compound IV offers the additional advantage of a considerably higher total yield at IIIb by a simpler and more effective purification in the final stage. The total yield at IIIb is 77%, around 7% higher than according to the Ru-catalyzed oxidation process and subsequent water elimination and even around 21 % higher than according to the one-pot process according to EP 075189 (Tab. 1).
As an alternative, Ib can be oxidized to IIb and converted directly to IIIb in the same pot by the reaction mixture being worked up under acidic conditions at pH < 5.

**Tab. 1: Comparison of the Yields of the Process According to the Invention Compared to the Process of the Prior Art**

| Process | **Yield (% of Theory)** | | |
|---|---|---|---|
| | **Ib → IIb** | **IIb → IIIb** | **Total (Ia→IIIb)** |
| Process According to the Invention | 82 (in the form of IV) | 94 | 77 |
| Ru-Catalyzed Oxidation According to EP 918791 | 75 | 94 | 70 |
| CrO₃ Oxidation According to EP 075189* | not isolated | not isolated | 56 |

| | | | |
|---|---|---|---|
| * See Table on page 7 EP 918791 | | | |

This invention is explained in more detail based on the examples below, without being limited thereto.

### Production Process

### General Operating Procedure 1 (GOP1): Synthesis of Compounds of Formula II

76.9 mmol of a compound of formula I is dissolved or suspended in 135 ml of dichloromethane. First, 0.15 g (1 mmol) of TEMPO is added to the mixture at 15°C. The addition of a solution that consists of 134 g of a 15.25% aqueous sodium hypochlorite solution (230.7 mmol) and 8.20 g (82 mmol) of potassium bicarbonate in 114 ml of water is carried out, whereby a pH-value of 9.1 is set. After the reaction is completed, the excess oxidizing agent is quenched at 15°C by adding an aqueous solution that consists of 12.5 g (76.5 mmol) of sodium phosphate and 10.6 g (55.8 mmol) of sodium disulfite (Na₂S₂O₅ and 121 ml of water.

The product of formula II is isolated from the organic phase by being precipitated from the reaction solution by adding 240 ml of diisopropyl ether, continuing to be stirred for 3 hours at 25°C, being filtered off and dried. As an alternative, the product that is already partially precipitated during the reaction depending on solubility in dichloromethane can be dissolved again by adding dichloromethane, and the organic phase is separated and redistilled in diisopropyl ether. The product that is precipitated in this case is filtered off with 300 ml of water, washed and dried.

### General Operating Procedure 2 (GOP2): Synthesis of Compounds of Formula III in a One-pot Process

76.9 mmol of a compound of formula I is dissolved or suspended in 135 ml of dichloromethane. First, 0.15 g (1 mmol) of TEMPO is added at 15°C to the mixture. The addition of a solution that consists of 134 g of a 15.25% aqueous sodium hypochlorite solution (230.7 mmol) and 8.20 g (82 mmol) of potassium bicarbonate in 114 ml of water is carried out, whereby a pH-value of 9.1 is set. After the reaction is completed, the excess oxidizing agent is quenched at 15°C by adding an aqueous solution of 10.6 g (55.8 mmol) of sodium disulfite (Na₂S₂O₅ in 121 ml of water.

The pH of the reaction solution is set at pH < 5 by adding dilute, aqueous sulfuric acid, and stirring is continued at room temperature until the reaction is complete.

The isolation of the product of formula III is carried out analogously to the isolation of the compounds of formula II according to GOP1, whereby the neutral washed organic phase is redistilled on diisopropyl ether. The product that is precipitated in this case is filtered off, washed with 300 ml of water and dried.

### General Operating Procedure 3 (GOP3): Synthesis of Compounds of Formula III Starting from Compounds of Formula II, in which R⁵ = OH:

0.1 mol of a compound of formula II, in which R⁵ = OH, obtained according to GOP1, is suspended in 65 ml of tetrahydrofuran or dioxane and acidified to a pH of 1 by adding 5 ml of 20% sulfuric acid. At room temperature, stirring of the reaction mixture is continued until dehydration is completed.

The isolation of the product of formula III is carried out by precipitation by means of the addition of 90 ml of water. The precipitated product is filtered off with water, washed neutral and dried.

### Example 1 6β,7β;15β,16β-Dimethylene-3-oxo-17α-pregnan-5β-ol-21,17-carbolactone-dichloromethane hemisolvate (IV):

According to GOP1, 30 g (0.0769 mol) of 17α-(3-hydroxypropyl)-6β,7β;15β,16β-dimethylene-androstane-3β,5β,17β-triol is reacted.

During the reaction, the product 6β,7β;15β,16β-dimethylene-3-oxo-17α-pregnan-5β-ol-21,17-carbolactone accumulates in the form of its dichloromethane hemisolvate. After excess oxidizing agent is destroyed and after working-up according to GOP1, 27 g of 6β,7β;15β,16β-dimethylene-3-oxo-17a-pregnan-5α-ol-21,17-carbolactone-dichloromethane hemisolvate (0.0630 mol) = 82% of theory is isolated.
[α]_{D}²⁰ = -61 ° (c = 1.0; CHCl₃); melting point = 121 °C;
¹H-NMR (400 MHz, CDCl₃): δ = 0.52 (q J = 5.5 Hz, 1H, 21 α-H [of the 15,16-methylene bridge]), 0.68-0.78 (m, 2H, 20-H [of the 6,7-methylene bridge]), 0.89-0.97 (m, 1H, 6-H), 0.93 (s, 3H, 19-H), 0.99 (s, 3H, 18-H), 1.19-1.52 (m, 7H), 1.54-1.85 (m, 6H), 1.92 (dd J = 3.8 and 11.8 Hz, 1H, 14-H), 2.06-2.16 (m, 1H, 22-H), 2.17-2.27 (m, 1H, 2α-H),2.32-2.69 (m, 5H), 2.96 (d J = 15.6 Hz, 1H, 4α-H), 5.30 (s, 1H, CH₂Cl₂).
¹³C-NMR (400 MHz, CDCl₃): δ = 9.97 (CH₂, C-21), 11.63 (CH₂, C-20), 16.74 (CH, C-15), 16.79 (CH, C-7), 17.29 (CH₃, C-19), 19.83 (CH₃, C-18), 21.75 (CH₂, C-11), 24.31 (CH, C-16), 24.76 (CH, C-6), 29.35 (CH₂, C-23), 30.70 (CH₂, C-22), 33.96 (CH, C-8), 34.47 (CH₂, C-1), 36.26 (CH₂, C-2), 37.31 (CH₂, C-12), 40.25 (C, C-10), 41.81 (C, C-13), 47.59 (CH, C-9), 52.18 (CH, C-14), 53.44 (CH₂Cl₂), 53.48 (CH₂, C-4), 75.57 (C, C-5), 96.24 (C, C-17), 176.63 (C, C-24), 210.56 (C, C-3).
MS (EI, 70eV) m/e = 384 (M⁺); m/e = 366 (M⁺-H₂O); m/e = 314 (M⁺-C₄H₆O); m/e = 111 (C₇C₁₁O⁺); m/e = 91 (C₆H₁₁O⁺); m/e = 55 (C₃H₃O⁺); m/e = 43 (C₂H₃O⁺).
IR: ϑ = 3483 cm⁻¹ (OH); ϑ = 1757 cm⁻¹ (C=O, lactone); ϑ = 1708 cm⁻¹ (C=O); ϑ = 1200 cm⁻¹ (O-C=O); ϑ = 1011 cm⁻¹ (C-O)

### Example 2 6β,7β;15β,16β-Dimethylene-3-oxo-17α-preg-4-ene-21,17-carbolactone (IIIb):

According to GOP2, 30 g (0.0769 mol) of 17α-(3-hydroxypropyl)-6β,7β;15β,16β-dimethylene-androstane-3β,5β,17β-triol is reacted. After excess oxidizing agent is destroyed according to GOP2, the reaction mixture is acidified with 10% sulfuric acid solution to a pH of 1 and stirred for 30 minutes at 25°C. After working-up according to GOP2, 21.5 g of 6β,7β;15β,16β-dimethylene-3-oxo-17α-preg-4-ene-21,17-carbolactone (0.059 mol) = 76.7% of theory is isolated.
[α]_{D}²² ≈ -182° (c = 0.5 CHCl₃); melting point = 201.3°C; UV (MeOH): ε₂₆₅ = 19,000; most important ¹H-NMR data (CDCl₃): δ = 0.40-0.67 (m, 1H, cyclopropyl H), 1.01 (s, 3H, 18-H), 1.11 (s, 3H, 19-H), 6.04 (s, 1H, 4-H) (D. Bittler, H. Hofmeister, H. Laurent, K. Nickisch, R. Nickolson, K. Petzoldt, R. Wiechert; Angew. Chem. Int. Ed. Engl. 1982, 21, 696-697];
MS (EI, 70eV) m/e = 366 (M⁺); m/e = 338 (M⁺-CO); m/e = 351 (M⁺-CH₃); significant fragments: m/e = 111; m/e = 136; m/e = 199, m/e = 217; m/e = 242; m/e = 255; m/e = 268; m/e = 293 [Interpretation: See W. Krause, G. Kuehne; Steroids 1982, 40, 81-90].

### Example 3 6β,7β:15β,16β-dimethylene-3-oxo-17α-preg-4-ene-21,17-carbolactone (IIIb):

According to GOP3, 30 g (70,25 mmol) of 6β,7β:15β,16β-dimethylene-3-oxo-17α-pregnane-5β-ol-21,17-carbolactone dichlormethane hemisolvat (from Example 1) is reacted to yield 24.30 g of drospirenone (yield: 94.5%).

## Claims

1. A process for the production of a 3-oxo-pregn-4-ene-21,17-carbolactones of formula IIIa 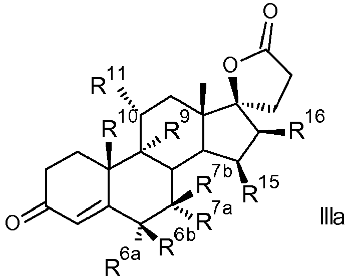 in which
R^{6a} is hydrogen, or together with R^{7a} a -CH₂ group;
R^{6b} is hydrogen, or together with R^{7b} a -CH₂ group, or a double bound;
R^{7a} is hydrogen, , C₁-C₄-alkoxycarbonyl, C₁-C₄-thioacyl,
R^{6b} is hydrogen, or together with R^{6b} a -CH₂ group;
R⁹ is hydrogen, together with R¹¹ a double bond, or together with R¹¹ an epoxy group -O-;
R¹⁰ is hydrogen, or methyl;
R¹¹ is hydrogen, together with R⁹ a double bond or together with R⁹ an epoxy group -O-;
R¹⁵ is hydrogen, together with R¹⁶ a -CH₂ group or a double bond;
R¹⁶ is hydrogen, together with R¹⁵ a -CH₂ group or a double bond;
comprising the oxidation of a compound of formula la 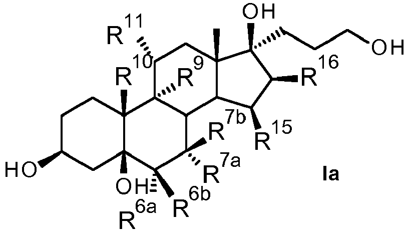 in which
R^{6a}, R^{6b}, R^{7a}, R^{7b} R⁹, R¹⁰, R¹¹, R¹⁵, R¹⁶ have the same meaning as in formula IIIa, to form a compound of formula IIa; 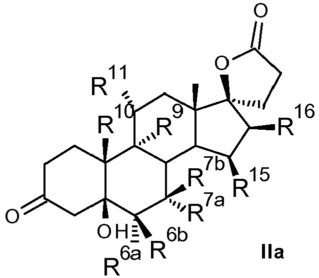 and the subsequent elimination of water with a suitable acid to form the compound of formula IIa;
**characterized in that**
the oxidation is performed with at least 3 molar equivalents of alkali hypochlorite, organic hypochlorite or 2-3 molar equivalents of alkaline-earth hypochlorite as an oxidizing agent in the presence of catalytic amounts of a 2,2,6,6-tetramethylpiperidine-N-oxide derivative at a pH of at least 8.0, in a two-phase solvent-water mixture, whereby the solvent is selected such that both the TEMPO derivative and the compounds of formula la can be well dissolved therein.

2. Process according to claim 1 for the production of a compound of formula IIIb 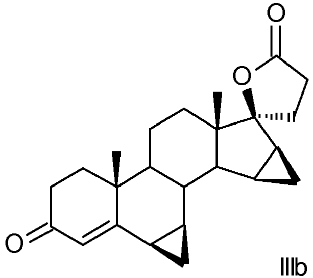 comprising the oxidation of the compound of formula Ib; 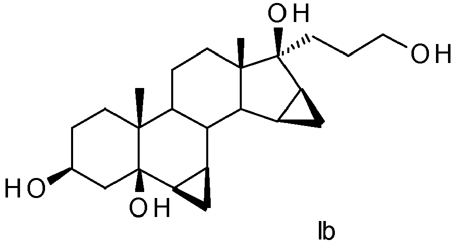 to form the compound of formula IIb, 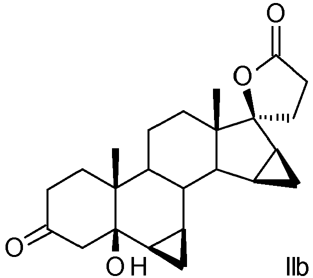 and the subsequent elimination of water to form the compound of formula IIIb.

3. A process for the production of a 3-oxo-pregn-4-ene-21,17-carbolactones of formula Ila 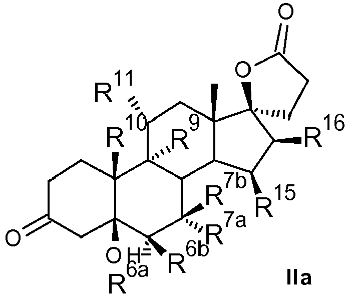 in which
R^{6a} is hydrogen, or together with R^{7a} a -CH₂ group;
R^{6b} is hydrogen, or together with R^{7b} a -CH₂ group, or a double bound;
R^{7a} is hydrogen, C₁-C₄-alkoxycarbonyl, C₁-C₄-thioacyl,
R^{6b} is hydrogen, or together with R^{6b} a -CH₂ group;
R⁹ is hydrogen, together with R¹¹ a double bond, or together with R¹¹ an epoxy group -O-;
R¹⁰ is hydrogen, or methyl;
R¹¹ is hydrogen, together with R⁹ a double bond or together with R⁹ an epoxy group -O-;
R¹⁵ is hydrogen, together with R¹⁶ a -CH₂ group or a double bond;
R¹⁶ is hydrogen, together with R¹⁵ a -CH₂ group or a double bond;
comprising the oxidation of a compound of formula la 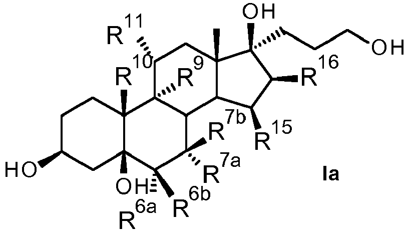 in which
R^{6a}, R^{6b}, R^{7a}, R^{7b}, R⁹, R¹⁰, R¹¹, R¹⁵, R¹⁶ have the same meaning as in formula IIa,
**characterized in that**
the oxidation is performed with at least 3 molar equivalents of alkali hypochlorite, organic hypochlorite or 2-3 molar equivalents of alkaline-earth hypochlorite as an oxidizing agent in the presence of catalytic amounts of a 2,2,6,6-tetramethylpiperidine-N-oxide derivative at a pH of at least 8.0, in a two-phase solvent-water mixture, whereby the solvent is selected such that both the TEMPO derivative and the compounds of formula la can be well dissolved therein.

4. Process according to claim 3 for the production of a compound of formula IIb 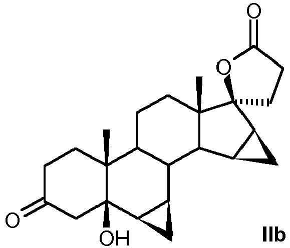 comprising the oxidation of the compound of formula Ib;

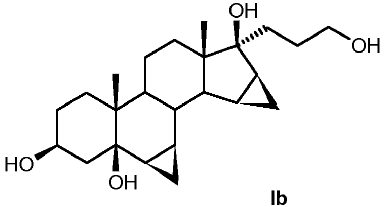

5. A process according to any one of the claims 1 to 4 in which the oxidation is performed in a dichloromethane-water mixture.

6. A process according to any one of the preceding claims, wherein 1-5 mol% of the 2,2,6,6-tetramethylpiperidine-N-oxide derivative is used.

7. A process according to any one of the preceding claims, wherein 1-1.5 mol% of 2,2,6,6-tetramethylpiperidine-N-oxide is used.

8. A process according to any one of the preceding claims, wherein 3-6 molar equivalents of alkali hypochlorite are used.

9. A process according to any one of the preceding claims, wherein 3-4 molar equivalents of sodium hypochlorite are used.

10. A process according to any one of the preceding claims, wherein the pH of the reaction solution is between 8.5 and 10.0.

11. A process according to any one of the preceding claims, wherein the pH of the reaction solution is set with potassium bicarbonate.

12. A process according to any one of the preceding claims, wherein the reaction temperature is 0 to 15°C.

13. A process according to any one of the preceding claims, wherein the compound of formula IIa or IIb is isolated by precipitation by means of addition of isopropylether.

14. A process according to any one of the preceding claims, wherein after the oxidation reaction is completed, a reducing agent for quenching excess hypochlorite reagent is added to the reaction mixture.

15. A process according to claim 14, wherein the reducing agent is added with the addition of a base or a basic buffer at a pH of more than 5.

16. A process according to any one of claims 14 or 15, wherein an aqueous alkali hydrogen sulfite solution is used as a reducing agent.

17. A process according to any one of claims 14 to 16, wherein as a reducing agent, sodium hydrogen sulfite or potassium hydrogen sulfite is used in the form of the aqueous solution of sodium disulfite or potassium disulfite.

18. A process according to any one of claims 15 to 17, wherein sodium phosphate (Na₃PO₄) is used as a base or a basic buffer.

## Patentansprüche

1. Verfahren zur Herstellung von 3-Oxo-17α-pregn-4-en-21,17-carbolactonen der Formel IIIa 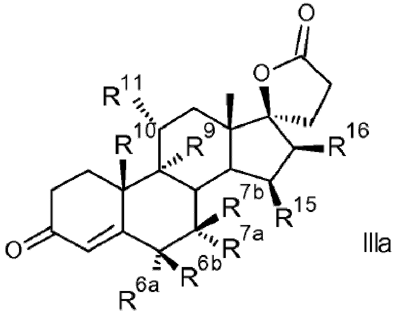 worin
R^{6a} Wasserstoff, oder zusammen mit R^{7a} eine -CH₂-Gruppe;
R^{6b} Wasserstoff, oder zusammen mit R^{7b} eine -CH₂-Gruppe oder eine Doppelbindung;
R^{7a} Wasserstoff, C₁-C₄-Alkoxycarbonyl, C₁-C₄-Thioacyl;
R^{7b} Wasserstoff, oder zusammen mit R^{6b} eine -CH₂-Gruppe;
R⁹ Wasserstoff, zusammen mit R¹¹ eine Doppelbindung oder zusammen mit R¹¹ eine Epoxygruppe -O-;
R¹⁰ Wasserstoff, oder Methyl;
R¹¹ Wasserstoff, zusammen mit R⁹ eine Doppelbindung oder zusammen mit R⁹ eine Epoxygruppe -O-;
R¹⁵ Wasserstoff, zusammen mit R¹⁶ eine -CH₂-Gruppe oder eine Doppelbindung;
R¹⁶ Wasserstoff, zusammen mit R¹⁵ eine -CH₂-Gruppe oder eine Doppelbindung
bedeuten,
umfassend die Oxidation einer Verbindung der Formel Ia 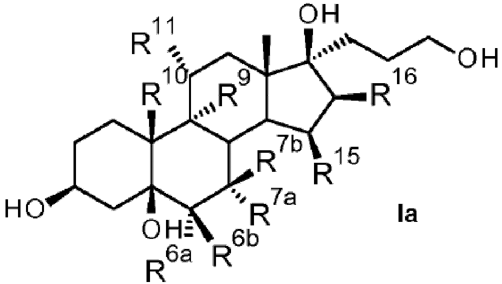 worin
die Reste R^{6a}, R^{6b}, R^{7a}, R^{7b}, R⁹, R¹⁰, R¹¹, R¹⁵, R¹⁶ dieselbe Bedeutung haben wie in Formel IIIa, unter Bildung einer Verbindung der Formel IIa 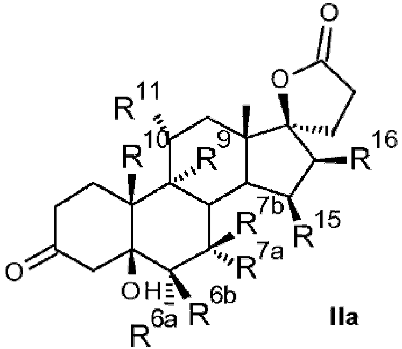 und nachfolgende Eliminierung von Wasser mit einer geeigneten Säure unter Bildung der Verbindung der Formel IIa;
**dadurch gekennzeichnet, dass**
die Oxidation mit mindestens 3 Moläq. Alkalihypochlorit, organisches Hypochlorit bzw. 2-3 Moläq. Erdalkalihypochlorit als Oxidationsmittel in Gegenwart von katalytischen Mengen eines 2,2,6,6-Tetramethylpiperidin-N-oxid-Derivates bei einem pH-Wert von mindestens 8,0, in einem zweiphasigen Lösungsmittel-Wasser-Gemisch durchgeführt wird, wobei das Lösungsmittel so gewählt wird, dass sich darin sowohl das TEMPO-Derivat als auch die Verbindungen der Formel Ia gut lösen.

2. Verfahren nach Anspruch 1 zur Herstellung einer Verbindung der Formel IIIb, 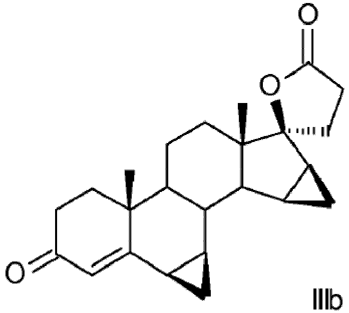 umfassend die Oxidation der Verbindung der Formel Ib 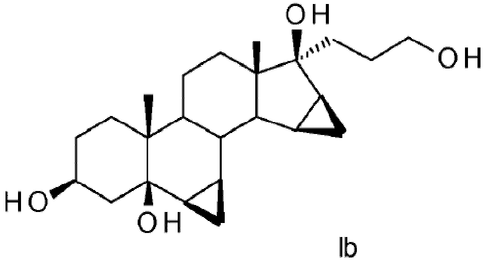 unter Bildung einer Verbindung der Formel IIb 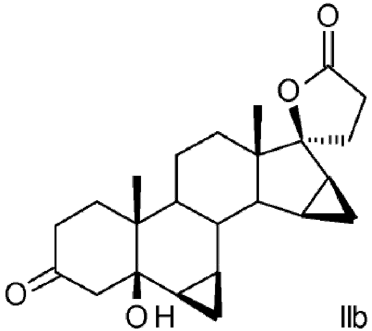 und nachfolgende Eliminierung von Wasser unter Bildung der Verbindung der Formel IIIb.

3. Verfahren zur Herstellung von 3-Oxo-17α-pregn-4-en-21,17-carbolactonen der Formel IIa 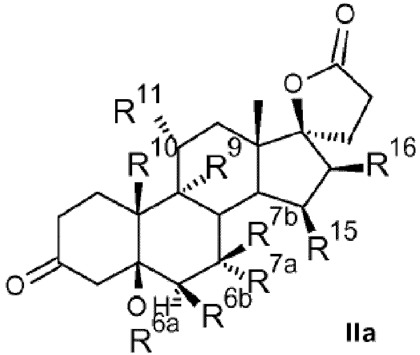 worin
R^{6a} Wasserstoff, oder zusammen mit R^{7a} eine -CH₂-Gruppe;
R^{6b} Wasserstoff, oder zusammen mit R^{7b} eine -CH₂-Gruppe oder eine Doppelbindung;
R^{7a} Wasserstoff, C₁-C₄-Alkoxycarbonyl, C₁-C₄-Thioacyl;
R^{7b} Wasserstoff, oder zusammen mit R^{6b} eine -CH₂-Gruppe;
R⁹ Wasserstoff, zusammen mit R¹¹ eine Doppelbindung oder zusammen mit R¹¹ eine Epoxygruppe -O-;
R¹⁰ Wasserstoff, oder Methyl;
R¹¹ Wasserstoff, zusammen mit R⁹ eine Doppelbindung oder zusammen mit R⁹ eine Epoxygruppe -O-;
R¹⁵ Wasserstoff, zusammen mit R¹⁶ eine -CH₂-Gruppe oder eine Doppelbindung;
R¹⁶ Wasserstoff, zusammen mit R¹⁵ eine -CH₂-Gruppe oder eine Doppelbindung
bedeuten,
umfassend die Oxidation einer Verbindung der Formel Ia 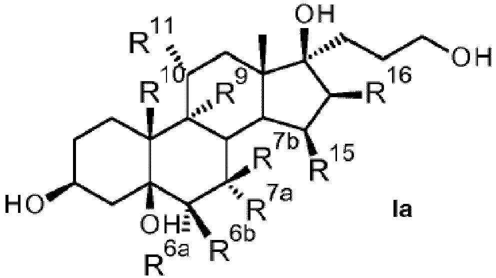 worin
die Reste R^{6a}, R^{6b}, R^{7a}, R^{7b}, R⁹, R¹⁰, R¹¹, R¹⁵, R¹⁶ dieselbe Bedeutung haben wie in Formel IIa,
**dadurch gekennzeichnet, dass**
die Oxidation mit mindestens 3 Moläq. Alkalihypochlorit, organisches Hypochlorit bzw. 2-3 Moläq. Erdalkalihypochlorit als Oxidationsmittel in Gegenwart von katalytischen Mengen eines 2,2,6,6-Tetramethylpiperidin-N-oxid-Derivates bei einem pH-Wert von mindestens 8,0, in einem zweiphasigen Lösungsmittel-Wasser-Gemisch durchgeführt wird, wobei das Lösungsmittel so gewählt wird, dass sich darin sowohl das TEMPO-Derivat als auch die Verbindungen der Formel Ia gut lösen.

4. Verfahren nach Anspruch 3 zur Herstellung einer Verbindung der Formel IIb, 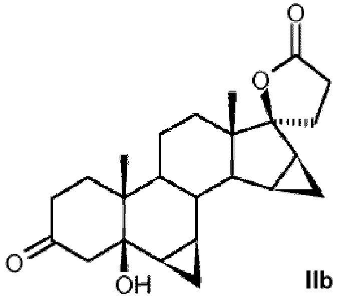 umfassend die Oxidation der Verbindung der Formel Ib

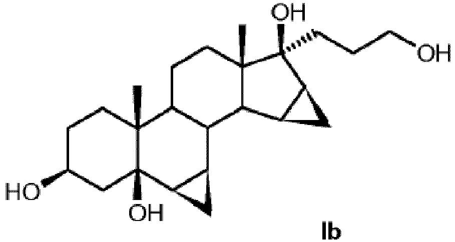

5. Verfahren nach einem der Ansprüche 1 bis 4, wobei die Oxidation in einer Dichlormethan-Wasser-Mischung durchgeführt wird.

6. Verfahren nach einem der vorherigen Ansprüche, wobei 1-5 mol% des 2,2,6,6-Tetramethylpiperidin-N-oxid-Derivates eingesetzt werden.

7. Verfahren nach einem der vorherigen Ansprüche, wobei 1-1,5 mol% 2,2,6,6-Tetramethylpiperidin-N-oxid eingesetzt werden.

8. Verfahren nach einem der vorherigen Ansprüchen, wobei 3-6 Moläq. Alkalihypochlorit eingesetzt werden.

9. Verfahren nach einem der vorherigen Ansprüche, wobei 3-4 Moläq. Natriumhypochlorit eingesetzt werden.

10. Verfahren nach einem der vorherigen Ansprüche, wobei der pH-Wert der Reaktionslösung zwischen 8,5 und 10,0 liegt.

11. Verfahren nach einem der vorherigen Ansprüche, wobei der pH-Wert der Reaktionslösung mit Kaliumhydrogencarbonat eingestellt wird.

12. Verfahren nach einem der vorherigen Ansprüche, wobei die Reaktionstemperatur 0 bis 15°C beträgt.

13. Verfahren nach einem der vorherigen Ansprüche, wobei die Verbindung der Formel IIa oder IIb durch Fällung durch Zugabe von Isopropylether isoliert wird.

14. Verfahren nach einem der vorherigen Ansprüche, wobei nach beendeter Oxidationsreaktion dem Reaktionsgemisch ein Reduktionsmittel zur Vernichtung des überschüssigen Hypochlorit-Reagenzes zugesetzt wird.

15. Verfahren nach Anspruch 14, wobei das Reduktionsmittel unter Zusatz einer Base oder eines basischen Puffers bei einem pH-Wert von größer als 5 zugesetzt wird.

16. Verfahren nach Anspruch 14 oder 15, wobei als Reduktionsmittel eine wässrige Alkalihydrogensulfit-Lösung verwendet wird.

17. Verfahren nach einem der Ansprüche 14 bis 16, wobei als Reduktionsmittel Natriumhydrogensulfit oder Kaliumhydrogensulfit in Form der wässrigen Lösung von Natriumdisulfit oder Kaliumdisulfit verwendet wird.

18. Verfahren nach einem der Ansprüche 15 bis 17, wobei als Base bzw. basischer Puffer Natriumphosphat (Na₃PO₄) verwendet wird.

## Revendications

1. Procédé pour la préparation de 3-oxo-4-prégnène-21,17-carbolactones de formule IIIa 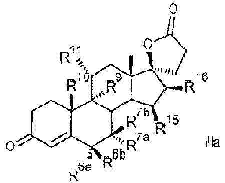 dans laquelle
R6a représente un atome d'hydrogène ou, conjointement avec R7a, un groupe -CH2 ;
R6b représente un atome d'hydrogène ou, conjointement avec R7b, un groupe -CH2 ou une double liaison ;
R7a représente un atome d'hydrogène, un groupe alcoxy(C1-C4)carbonyle, thioacyle en C1-C4,
R7b représente un atome d'hydrogène ou, conjointement avec R6b, un groupe -CH2 ;
R9 représente un atome d'hydrogène ou, conjointement avec R11, une double liaison ou, conjointement avec R11, un groupe époxy -0- ;
R¹⁰ représente un atome d'hydrogène, ou un groupe méthyle ;
R11 représente un atome d'hydrogène ou, conjointement avec R9, une double liaison ou, conjointement avec R9, un groupe époxy -0- ;
R15 représente un atome d'hydrogène, conjointement avec R16, un groupe -CH2 ou une double liaison ;
R16 représente un atome d'hydrogène ou, conjointement avec R15, un groupe -CH2 ou une double liaison ;
comprenant l'oxidation d'un compose de formule Ia 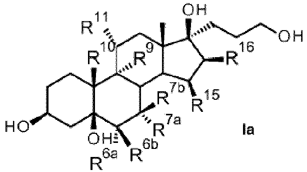 dans laquelle
les radicaux R6a, R6b, R7a, R7b, R9, R10, R11, R15, R16 ont les mêmes significations que dans la formule IIIa, pour former un compose de formule IIa; 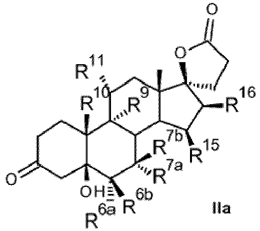 avec élimination subséquente d'eau avec un acide approprié pour former le composé de formule IIa ; caractérisé en se que l'oxdation est réalisé avec au moins 3 équivalents molaires d'un hypochlorite de métal alcalin, d'un hypochlorite organique, ou
2-3 équivalents molaires d'un hypochlorite de métal alcalino-terreux en tant qu'oxydant, en présence de quantités catalytiques d'un dérivé de 2,2,6,6-tétraméthylpipéridine-N-oxyde, à un pH d'au moins 8,0, dans un mélange biphasique de solvant-eau cependant le solvant sélecté est tel que le dérivate TEMPO ainsi que les composés de formule la peuvent être bien dissous dedans

2. Procédé selon la revendication 1, pour la préparation du composé de formule IIIb 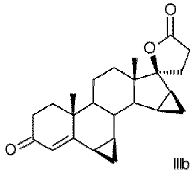 comprenant l'oxydation du composé de formule Ib; 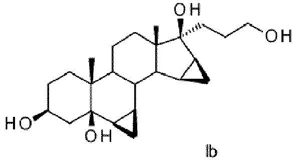 pour former le composé de formule IIb, 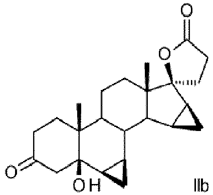 avec élimination subséquente d'eau pour former le composé de formule IIb.

3. Procédé pour la préparation de 3-oxo-4-prégnène-21,17-carbolactones de formule IIa 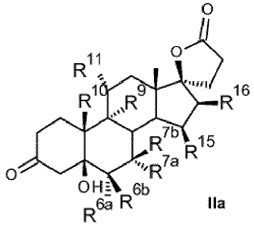 dans laquelle
R6a représente un atome d'hydrogène ou, conjointement avec R7a, un groupe -CH2 ;
R6b représente un atome d'hydrogène ou, conjointement avec R7b, un groupe -CH2 ou une double liaison ;
R7a représente un atome d'hydrogène, un groupe alcoxy(C1-C4)carbonyle, thioacyle en C1-C4,
R7b représente un atome d'hydrogène ou, conjointement avec R6b, un groupe -CH2 ;
R9 représente un atome d'hydrogène ou, conjointement avec R11, une double liaison ou, conjointement avec R11, un groupe époxy -0- ;
R10 représente un atome d'hydrogène, ou un groupe méthyle ;
R11 représente un atome d'hydrogène ou, conjointement avec R9, une double liaison ou, conjointement avec R9, un groupe époxy -0- ;
R15 représente un atome d'hydrogène, conjointement avec R16, un groupe -CH2 ou une double liaison ;
R16 représente un atome d'hydrogène ou, conjointement avec R15, un groupe -CH2 ou une double liaison ;
comprenant
l'oxidation d'un composé de formule Ia 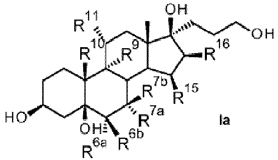 dans laquelle
les radicaux R6a, R6b, R7a, R7b, R9, R10, R11, R15, R16 ont les mêmes significations que dans la formule IIa, **caractérisé en ce que**,
l'oxydation est réalisée avec au moins 3 équivalents molaires d'un hypochlorite de métal alcalin, d'un hypochlorite organique, ou au 2-3 équivalents molaires d'un hypochlorite de métal alcalino-terreux en tant qu'oxydant, en présence de quantités catalytiques d'un dérivé de 2,2,6,6-tétraméthylpipéridine-N-oxyde, à un pH d'au moins 8,0, dans un mélange biphasique de solvant-eau cependant le solvant sélecté est tel que le dérivate TEMPO ainsi que les composés de formule la peuvent être bien dissous dedans

4. Procédé selon revendication 3 pour la préparation du composé de formule IIb : 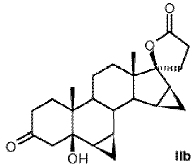 comprenant l'oxydation d'u composé de formule Ib ;

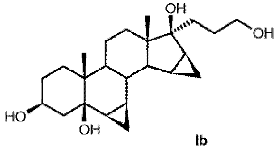

5. Procéde selon l'une quelconque des revendications 1 à 4, dans lequel l'oxydation est réalisée dans un mélange dichlorométhane/eau.

6. Procédé selon l'une quelconque des revendications précédentes, **caractérisé en ce qu'**on utilise 1-5 % en moles du dérivé de 2,2,6,6-tétraméthylpipéridine-N-oxyde.

7. Procédé selon l'une quelconque des revendications précédentes, **caractérisé en ce qu'**on utilise 1-1,5% en moles de 2,2,6,6-tétraméthylpipéridine-N-oxyde.

8. Procédé selon l'une quelconque des revendications précédentes, **caractérisé en ce qu'**on utilise 3-6 équivalents molaires d'un hypochlorite de métal alcalin.

9. Procédé selon l'une quelconque des revendications précédentes, **caractérisé en ce qu'**on utilise 3-4 équivalents molaires d'hypochlorite de sodium.

10. Procédé selon l'une quelconque des revendications précédentes, **caractérisé en ce que** pH de la solution réactionnelle est compris entre 8,5 et 10,0.

11. Procédé selon l'une quelconque des revendications précédentes, **caractérisé en ce que** le pH de la solution réactionnelle est ajusté à l'aide d'hydrogénocarbonate de potassium.

12. Procédé selon l'une quelconque des revendications précédentes, **caractérisé en ce que** la température de la réaction est dans la plage de 0 à 15 °C.

13. Procédé selon l'une quelconque des revendications précédentes, **caractérisé en ce que** le composé de formule IIa ou IIb est isolé par précipitation en ajoutant de l'éther isopropylique.

14. Procédé selon l'une quelconque des revendications précédentes, **caractérisé en ce qu'**une fois terminée la réaction d'oxydation on ajoute au mélange réactionnel un réducteur pour détruire le réactif hypochlorite en excès.

15. Procédé selon la revendication 14, **caractérisé en ce qu'**on ajoute le réducteur à un pH de plus de 5 par addition d'une base ou d'un tampon basique.

16. Procédé selon la revendication 14 ou 15, **caractérisé en ce qu'**on utilise comme réducteur une solution aqueuse d'hydrogénosulfite de métal alcalin.

17. Procédé selon l'une quelconque des revendications 14 à 16, **caractérisé en ce qu'**on utilise omme réducteur de l'hydrogénosulfite de sodium ou de l'hydrogénosulfite de potassium sous forme de la solution aqueuse de disulfite de sodium ou de disulfite de potassium.

18. Procédé selon l'une quelconque des revendications 15 à 17, **caractérisé en ce qu'**on utilise comme base ou tampon basique du phosphate de sodium (Na3P04).
